# EUROPEAN PATENT APPLICATION

(11) **EP 2 266 578 A1**
(43) Veröffentlichungstag der Anmeldung: **29.12.2010**
(21) Anmeldenummer: 08873431.4
(22) Anmeldetag: 18.03.2008
(51) Int. Cl.: A61K 31/663, A61K 9/06, A61K 9/127, A61P 19/08, A61P 19/10

(54) **PHARMAZEUTISCHE ZUSAMMENSETZUNG ZUR PRÄVENTION UND BEHANDLUNG VON KNOCHENRESORPTION UNTERSCHIEDLICHER ÄTIOLOGIE**

(71) Anmelder: Dikovskiy, Aleksander Vladimirovich, Moscow 123182 (RU)
(72) Erfinder: DOROZHKO, Oleg Valentinovich, Moscow 115446 (RU); CHUBATOVA, Svetlana Aleksandrovna, Moskovskaya obl. 142279 (RU); RUDOI, Boris Anatolievich, Moscow 109518 (RU)
(74) Vertreter: Jeck, Anton
(86) Internationale Anmeldenummer: PCT/RU2008/000153
(87) Internationale Veröffentlichungsnummer: WO 2009/116888

(57) **Zusammenfassung**

Die Erfindung bezieht sich auf die Gebiete der Medizin und der Pharmakologie. Sie stellt eine pharmazeutische Zusammensetzung zur Behandlung von Knochenresorption unterschiedlicher Ätiologie dar und weist Liposomen mit Bisphosphonaten in Form eines Gels als biologisch aktive Wirkstoffe auf. Die pharmazeutische Zusammensetzung ist für eine transdermale Lieferung der Bisphosphonate und für eine wirksame Lokalbehandlung gut geeignet. Das vorgeschlagene Verfahren zur Vorbeugung und Behandlung von Osteoporose, Morbus Paget (Paget-Krankheit), pathologischen Frakturen bei Krebskrankheiten und manchen anderen pathologischen Vorgängen, die mit Knochenresorption und Wiederherstellung der Knochengewebe zusammenhängen, sieht den Einsatz der genannten Zusammensetzung vor.

## Beschreibung

Die Erfindung betrifft eine pharmazeutische Zusammensetzung in Form eines Gels zur Vorbeugung und Behandlung von Osteoporose und anderen Zuständen nach dem Oberbegriff des Anspruchs 1.

Die angemeldete Gruppe von Erfindungen betrifft das Gebiet der Medizin, nämlich Arzneimittel und Zusammensetzungen, die zur Vorbeugung und Behandlung von Knochenresorption und zwar Osteoporose, Morbus Paget (Paget-Krankheit), pathologischen Frakturen bei von Krebs betroffenen Kranken sowie zur Vorbeugung von Osteoporose im Klimakterium dienen.

Bei der Einführung in den Körper von warmblütigen Lebewesen zeigt Natriumalendronat seine Osteoselektivität auf. Dabei wirkt es mit dem Knochengewebe als ein spezifischer Inhibitor der osteoklastisch vermittelten Knochenresorption zusammen. Als synthetisches Vergleichserzeugnis von Pyrophosphat verbindet sich das Natriumalendronat auf eine spezifische Weise mit Hydroxyappatit des Knochens. Dies verhindert eine Resorption des Knochengewebes.

Zugleich weist Natriumalendronat als Arzneimittel wesentliche Nachteile auf. Darunter fallen z. B. eine äußerst geringe biologische Zugänglichkeit bei Intussuszeption (weniger als 1 %) und oftmals Komplikationen, die mit der Reizung und Geschwürbildung auf der Schleimhaut des Magen-Darm-Kanals zusammenhängen. Folglich kann Natriumalendronat bei vielen Kranken nicht verordnet werden, die von MDK-Erkrankungen betroffen sind.

Es ist allgemein anerkannt, dass der Eintritt von Bisphosphonaten in die Systemblutströmung (sogar bei intravenöser Injektion) keinen prinzipiellen Zeitpunkt bei der Behandlung von Osteoporose oder anderer Erkrankungen mit Knochenresorption darstellt. Die Hauptursache sind die Zulieferung, die Verteilung und die Exposition der Bisphosphonate auf der Oberfläche der porösen Knochen.

Bekanntlich können die Bisphosphonate beim Auftragen auf die Haut absorbiert werden. Die Verfahren zur Optimierung der Durchdringung der Salze der diphosphonischen Säuren durch die Haut wurden von Ferrini P.G. et al. vorgeschlagen (EP 0407344, veröffentlicht am 27.02.1991).

Dieses Verfahren hat folgenden Mangel: Die Bisphosphonate als Bestandteile der vorgeschlagenen Rezepturen dringen nur sehr schwer in den Unterhautfellzellstoff ein und werden schnell aus dem "Depot" eliminiert. Darüber hinaus werden zur Optimierung des transdermalen Transports zusätzliche Komponenten eingesetzt, die Hautreizungen oder allergische Reaktionen hervorrufen können.

Das Patent US 6905701 (veröffentlicht am 14.06.2005) schlägt ein Verfahren für lokale Applikation von Natriumalendronat vor. Gemäß diesem Verfahren wird Natriumalendronat als Bestandteil von Vaginalzäpfchen, weichen Kapseln oder Creme über die Schleimhaut der Scheide geliefert.

Jedoch weist dieses Verfahren wesentliche Mängel auf, die mit der Reizwirkung von Alendronat auf die Schleimhäute und mit den natürlichen Einschränkungen bei der Anwendung des Präparats je nach Geschlecht des Patienten zusammenhängen.

Aus dem Stand der Technik ist eine lokale Anwendung von Natriumalendronat zur Vorbeugung und Behandlung von Knochenresorption unterschiedlicher Ätiologie bekannt. Dabei wird der wirksame Bestandteil mittels lontophorese geliefert (US Patent 6 008 206, veröffentlicht am 28. Dezember 1999).

Der Mangel dieses Vorschlags besteht darin, dass die Zulieferung von Natriumalendronat nach dem lontophorese-Verfahren es ermöglicht, geringe Konzentrationen des Präparats an der Einführungsstelle zu erzielen. Deswegen bedarf es einer fachspezifischen medizinischen Ausrüstung.

Der nächste Stand der Technik gegenüber der vorgeschlagenen Erfindung ist seinem Wesen nach eine pharmazeutische Zusammensetzung zur Behandlung von Osteoarthritis und Osteoporose unterschiedlicher Ätiologie. Diese pharmazeutische Zusammensetzung enthält Natriumalendronat, Weißvaseline, Salizylalkohol, weißes Bienenwachs, Glyzerin, Paraffinöl, Natriumlaurylsulfat und Wasser. Der nächste Stand der Technik ist auch ein Verfahren zur lokalen Anwendung von Natriumalendronat. Es umfasst eine selektive Lieferung des Präparats an die Stellen mit erhöhter Knochengeweberesorption (US Patent 5 958 908, veröffentlicht am 28.09.1999).

Die Mängel dieser Zusammensetzung und des Anwendungsverfahrens bestehen darin, dass es nicht möglich ist, eine erforderliche Konzentration von Natriumalendronat in Geweben und Knochen unmittelbar im Bereich der Lokalisation des pathologischen Vorgangs sicherzustellen. Die Ursache ist eine zu schwache Durchdringungsfähigkeit des aktiven Stoffs als Bestandteil des durch die Erfinder vorgeschlagenen Grundstoffs.

Es ist Aufgabe der Erfindung, die vorgeschlagene Zusammensetzung bei der transdermalen Lieferung mit einer ausreichend hohen Konzentration an Natriumalendronat in Geweben und in Knochen unmittelbar an der Stelle der Applikation des Präparats auf die Haut sicherzustellen und zwar ohne dass dazu zusätzliche medizinische Ausrüstung oder technische Verfahren (Iontophorese, Elektrophorese, Einreibungen mittels Massagen, Injektionen des Präparats) eingesetzt werden müssen.

Die gestellte Aufgabe wird durch die Merkmale des Anspruchs 1 bzw. des Anspruchs 4 gelöst.

Die vorgeschlagenen Erfindungen sind durch einen einheitlichen Erfindungsgedanken zu einer Gruppe verbunden. Die Aufgabe der Gruppe von Erfindungen ist die Herstellung einer pharmazeutischen Zusammensetzung von Natriumalendronat in der Arzneimittelform von Gel und die Entwicklung eines Verfahrens zur Anwendung dieser Zusammensetzung für eine transdermale Applikation. Natriumalendronat oder andere Bisphosphonate stellen hier die Bestandteile von Liposomen dar. Die Liposome verbessern wesentlich die biologische Zugänglichkeit von Natriumalendronat und schaffen Bedingungen für eine andauernde lokale Erhaltung der therapeutischen Konzentrationen von Natriumalendronat in den mit Knochenresorption unterschiedlicher Ätiologie betroffenen Stellen, in den Bereichen der pathologischen Frakturen sowie bei anderen Zuständen, die einer Wiederherstellung ("Reparatur") von Knochengewebe bedürfen.

Das Wesen der Erfindung in Bezug auf die pharmazeutische Zusammensetzung in Form von Gel zur Vorbeugung und Behandlung von Osteoporose und anderen Zuständen, die mit der Notwendigkeit der Wiederherstellung der Knochengewebe zusammenhängen, ist wie folgt: Die pharmazeutische Zusammensetzung in Form von Gel enthält Bisphosphonat als wirksamen Bestandteil. Dabei ist das Bisphosphonat in Phospholipide-Bläschen eingegliedert, die aus lipide- und wasseraufnehmenden Phasen gebildet sind. Die lipide- und wasseraufnehmenden Phasen umfassen Komponenten in folgendem Komponentenverhältnis (Gew. %):

| | |
|---|---|
| Bisphosphonat | 0,01 - 2,0 |
| Albuminlezithin | 1,0 - 6,0 |
| ätherisches Kieferöl | 0,05 - 0,2 |
| Kampferöl | 0,01 - 1,0 |
| Olivenöl | 0,01 - 5,0 |
| Vitamin E | 0,01 - 0,15 |
| Vitamin D | 0,01 - 0,2 |
| Carbopol | 0,4 - 0,6 |
| NaOH | 0,42 |
| Glyzerin | 2,0 - 4,0 |
| Nipagin | 0,3 |
| Nipasol | 0,1 |
| Wasser | Rest. |

Das Bisphosphonat ist vorzugsweise aus folgender Gruppe gewählt: Alendronat, Risedronat, Etidronat, Clodronat, Pamidronat. Dabei liegen die Größen der bisphosphonathaltigen Phospholipide-Bläschen im Bereich von 50 bis 250 nm.

Das Wesen der Erfindung in Bezug auf das Verfahren zur Behandlung von Knochengeweberesorption unterschiedlicher Ätiologie besteht im Folgenden: Die oben genannte pharmazeutische Zusammensetzung in Form von Gel enthält Bisphosphonat als wirksamen Bestandteil. Dabei ist das Bisphosphonat in die Phospholipide-Bläschen eingegliedert. Die Phospholipide-Bläschen sind aus lipide- und wasseraufund wasseraufnehmenden Phasen gebildet. Die lipide- und wasseraufnehmenden Phasen umfassen Komponenten in folgendem Komponentenverhältnis (Gew. %):

| | |
|---|---|
| Bisphosphonat | 0,01 - 2,0 |
| Albuminlezithin | 1,0 - 6,0 |
| ätherisches Kieferöl | 0,05 - 0,2 |
| Kampferöl | 0,01 - 1,0 |
| Olivenöl | 0,01 - 5,0 |
| Vitamin E | 0,01 - 0,15 |
| Vitamin D | 0,01 - 0,2 |
| Carbopol | 0,4 - 0,6 |
| NaOH | 0,42 |
| Glyzerin | 2,0 - 4,0 |
| Nipagin | 0,3 |
| Nipasol | 0,1 |
| Wasser | Rest. |

Das Bisphosphonat ist vorzugsweise aus folgender Gruppe gewählt: Alendronat, Risedronat, Etidronat, Clodronat, Pamidronat. Dabei liegen die Größen der bisphosphonathaltigen Phospholipide-Bläschen im Bereich von 50 bis 250 nm.

Das Gel wird auf die Haut an den Stellen der durch Diagnose festgestellten Resorption oder der Destruktion des Knochengewebes aufgetragen.

Das Wesen der Erfindung in Bezug auf das Verfahren zur Behandlung von Osteoporose bei Patienten mit Pathologien der Organe des Magen-Darm-Kanals besteht im Folgenden. Die oben genannte pharmazeutische Zusammensetzung in Form von Gel wird transdermal angewendet. Die pharmazeutische Zusammensetzung in Form von Gel enthält Bisphosphonat als wirksamen Bestandteil. Es ist in die Phospholipide-Bläschen eingegliedert. Die Phospholipide-Bläschen sind aus Lipide- und wasseraufnehmenden Phasen gebildet. Diese Phasen umfassen Komponenten mit folgendem Komponentenverhältnis (Gew. %):

| | |
|---|---|
| Bisphosphonat | 0,01 - 2,0 |
| Albuminlezithin | 1,0 - 6,0 |
| ätherisches Kieferöl | 0,05 - 0,2 |
| Kampferöl | 0,01 - 1,0 |
| Olivenöl | 0,01 - 5,0 |
| Vitamin E | 0,01 - 0,15 |
| Vitamin D | 0,01 - 0,2 |
| Carbopol | 0,4 - 0,6 |
| NaOH | 0,42 |
| Glyzerin | 2,0 - 4,0 |
| Nipagin | 0,3 |
| Nipasol | 0,1 |
| Wasser | Rest. |

Das Bisphosphonat ist vorzugsweise aus folgender Gruppe gewählt: Alendronat, Risedronat, Etidronat, Clodronat, Pamidronat. Dabei liegen die Größen der bisphosphonathaltigen Phospholipide-Bläschen im Bereich von 50 bis 250 nm.

In diesem Fall schließt die transdermale Applikation von Gel Schädigungen der Schleimhaut von MDK aus, welche für eine Intussuszeption der Bisphosphonate kennzeichnend sind.

Die Erfindung wird anhand der in den Zeichnungen dargestellten Kennlinien näher erläutert. Es zeigen:

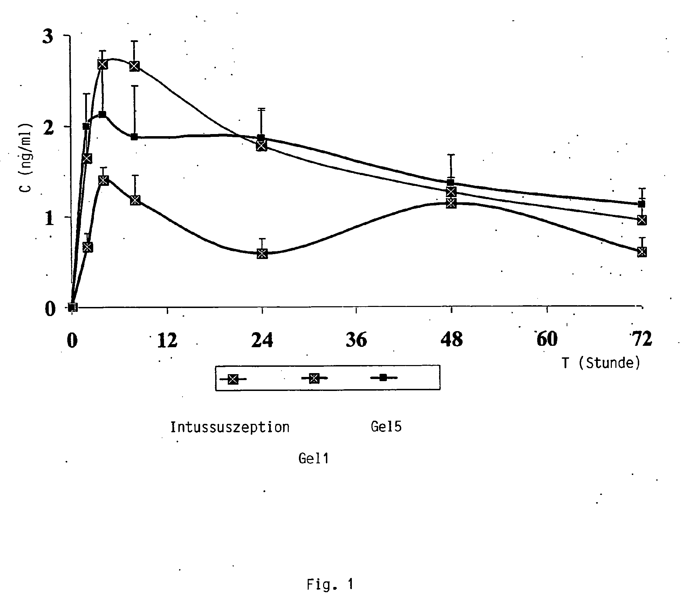

- Fig. 1: die kinetischen Kennlinien der Konzentrationen von Natriuma- lendronat im Plasma der untersuchten Weißratten nach einer In- tussuszeption von Natriumalendronat sowie nach einem ein- und fünfmaligen Auftragen als Bestandteil des Liposomen-Gels auf die Haut der inneren Schenkeloberfläche und

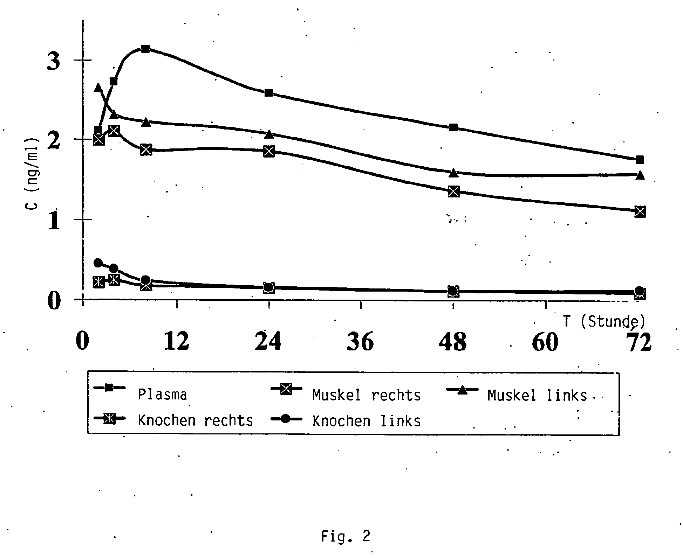

- Fig. 2: die kinetischen Kennlinien der Konzentrationen von Natriuma- lendronat im Blutplasma der Weißratten, im Muskelgewebe von Schenkel und im Oberschenkelknochen nach einem fünfmaligen Auftragen von Natriumalendronat auf die Haut. Dabei handelt es sich um das Natriumalendronat als Bestandteil des Liposomen- Gels. Das Gel wurde auf die innere Fläche des Schenkels der Ratten aufgetragen. Die Dosis betrug 1 mg (4,80 ± 0,34 mg/kg).

Die angemeldete pharmazeutische Zusammensetzung zur Vorbeugung und Behandlung von Knochenresorption unterschiedlicher Ätiologie weist als aktive Komponente Natriumalendronat auf. Das Natriumalendronat ist in Phospholipide-Bläschen eingegliedert, welche aus lipide- und wasseraufnehmenden Phasen gebildet sind. Dabei umfasst die Lipide-Phase: Albuminlezithin, ätherisches Kieferöl, Kampferöl und Olivenöl, Vitamine E, D, F. Die hydrophile Phase enthält Natriumalendronat. Die Zusammensetzung weist zusätzlich ein gelbildendes Mittel auf, welches Carbopol, Na-OH 10 %, einen Weichmacher - Glyzerin, Konservierungsstoffe - Nipagin, Nipasol und Wasser enthält. Das Komponentenverhältnis in der Zusammensetzung ist wie folgt (Gew. %):

| | |
|---|---|
| Natriumalendronat | 0,01 - 0,1 |
| Albuminlezithin | 1 - 6 |
| ätherisches Kieferöl | 0,05 - 0,2 |
| Kampferöl | 0,01 - 1 |
| Olivenöl | 0,01 - 5 |
| Vitamin E | 0,01 - 0,15 |
| Vitamin D | 0,01 - 0,2 |
| Vitamin F | 0,2 - 0,4 |
| Carbopol | 0,4 - 0,6 |
| NaOH | 0,42 |
| Glyzerin | 2 - 4 |
| Nipagin | 0,3 |
| Nipasol | 0,1 |
| Wasser | Rest. |

Dabei sind die Komponenten in einem hochdrehzahligen Homogenisator behandelt. Darin werden die Einschicht-Liposome mit bestimmten Dimensionen gebildet, die durch die Behandlung der fettlöslichen Zutaten hergestellt werden.

Das Ergebnis wird auch auf folgende Weise erreicht. Die pharmazeutische Zusammensetzung enthält Liposome mit Natriumalendronat als Bestandteil von Gel. Die pharmazeutische Zusammensetzung wird auf die Haut aufgetragen und zwar unmittelbar an den Stellen der durch Diagnose festgestellten Knochengeweberesorption (Frakturstellen, Gelenke, Wirbel usw.).

Der aktive Transport der Arzneimittel und der biologisch aktiven Substanzen als Bestandteile von Liposomen wurde bereits mehrmals nachgewiesen (z. B. Patent RU No. 2167650, BI Heft 15, 2001). Durch die Eingliederung in die Liposomen gelang es, nicht nur die Heilmittel in die Zielorgane zu liefern, sondern auch in vielen Fällen die erforderliche Konzentration der Heilmittel zu reduzieren. Das ist beim Einsatz der Heilmittel, die Nebenwirkungen aufweisen, sehr wichtig.

Das Ziel der vorgeschlagenen Erfindung ist die Herstellung von liposomenhaltigen pharmazeutischen Zusammensetzungen der Bisphosphonate für einen transdermalen Transport. Ihre aktiven Bestandteile können folgende Substanzen sein: Alendronat, Risedronat, Etidronat, Clodronat, Pamidronat usw. Ihr Einsatz wird in solchen Zuständen vorgeschlagen, welche mit der Resorption der Knochengewebe als Ergebnis Osteoporose sowie mit der Regeneration der Knochen bei pathologischen Frakturen, Knochenplastik u. a. m. zusammenhängen. Die vorgeschlagene pharmazeutische Zusammensetzung enthält ein gelbildendes Mittel, einen Weichmacher, die Lipide-Phase, die hydrophile Phase, Bisphosphonate, Konservierungsstoff, ätherisches Öl und Wasser. Diese Zusammensetzung ist durch folgende Beispiele beschrieben.

### Beispiel 4

| | |
|---|---|
| Natriumalendronat | 0,01 |
| Albuminlezithin | 2,0 |
| ätherisches Kieferöl | 0,05 |
| Kampferöl | 0,05 |
| Olivenöl | 1,0 |
| Vitamin E | 0,1 |
| Vitamin D | 0,1 |
| Vitamin F | 0,2 |
| Carbopol | 0,4 |
| NaOH | 0,42 |
| Glyzerin | 2,0 |
| Nipagin | 0,3 |
| Nipasol | 0,1 |
| Wasser | Rest |

### Beispiel 5

| | |
|---|---|
| Natriumalendronat | 0,05 |
| Carbopol | 0,5 |
| Glyzerin | 4,0 |
| NaOH | 0,42 |
| Albuminlezithin | 1,0 |
| Vitamin E | 0,05 |
| Vitamin D | 0,1 |
| Vitamin F | 0,2 |
| Kampferöl | 0,4 |
| Olivenöl | 2,0 |
| ätherisches Kieferöl | 0,1 |
| Nipagin | 0,3 |
| Nipasol | 0,1 |
| Wasser | Rest |

### Beispiel 6

| | |
|---|---|
| Natriumalendronat | 0,1 |
| Carbopol | 0,6 |
| Glyzerin | 4,0 |
| NaOH | 0,42 |
| Albuminlezithin | 3,0 |
| Olivenöl | 5,0 |
| Vitamin E | 0,15 |
| Vitamin D | 0,1 |
| Vitamin F | 0,2 |
| ätherisches Kieferöl | 0,2 |
| Nipagin | 0,3 |
| Nipasol | 0,1 |
| Wasser | Rest |

Das Natriumalendronat gemäß der angemeldeten pharmazeutischen Zusammensetzung ist in sehr kleine (von 50 bis 250 nm) Bläschen-Teilchen eingegliedert. Diese sind in einem System von Polymeren unterschiedlicher Natur stabilisiert. Somit wirkt die angemeldete pharmazeutische Zusammensetzung als Inhibitor der osteoklastisch ermittelten Knochenresorption und kann zur Korrektur der Knochenresorption bei Osteoporose von Frauen in der Postmenopause verwendet werden. Die Anwendung der vorgeschlagenen Zusammensetzung baut die Menge der Kompressionsbrüche von Wirbelsäule und Schenkelhals wesentlich ab und verringert die Porose (Leerraumanteil) der Knochen in gesetztem Alter sowohl bei Frauen als auch bei Männern.

Die liposomenhaltige Zusammensetzung mit Bisphosphonaten ist als Gel verwendbar und stellt eine hohe biologische Zugänglichkeit und einen aktiven Transport der Zusammensetzung in die tiefen Schichten des Unterhautzellstoffs, in das Muskelund Bindegewebe sowie in die umgebenden Knochen sicher.

Die transdermale Lieferung der Zusammensetzung erfolgt leicht dadurch, dass eine bestimmte Gelmenge auf die Haut gemäß der Projektion der durch die Diagnose festgestellten Porose der Knochen oder an den Stellen der Wiederherstellung der Knochengewebe nach Frakturen aufgetragen wird. Das Gel enthält dabei eine stabile Aufschwemmung von Liposomen.

Die Anwendung von Liposomen ermöglicht eine Optimierung der Konzentration der Aktivstoffe als Bestandteil des Gels. Dadurch ist ein maximaler therapeutischer Effekt erreicht, und der Transport der Bisphosphonate in den Bereich von bestimmten Wirbelsäuleabschnitten, in die Röhrenknochen, Gelenke und Gewebe, die die poröse bzw. verformte Knochen umringen, ist sichergestellt.

Die vorgeschlagene liposomenhaltige Zusammensetzung ermöglicht es, die Eigenschaften von Natriumalendronat oder anderer Bisphosphonate wirksam zu benutzen, um die zusätzlichen aktiven Wirkstoffe anzuwenden und den Phospholipidemangel in den mit Gel behandelten Stellen zu beseitigen.

Das Verfahren zur Lieferung von Natriumalendronat an die Stellen mit Knochenresorption besteht in der Behandlung der jeweiligen Körperbereiche mit bestimmten Mengen von liposomen- und bisphosphonatehaltigem Gel. Das Gel wird auf die Haut gemäß der Projektion der Knochengewebefehler, der Kompressionsbrüche von Wirbelsäule und Schenkelhals oder in den Bereichen der Wiederherstellung von Knochengewebe bei der Knochenbruchtherapie oder bei der Knochenplastik aufgetragen.

Die Zusammensetzung wird folgenderweise hergestellt:

Die einzuführenden Komponenten der pharmazeutischen Zusammensetzung werden zuerst gemäß der oben beschriebenen Rezeptur (Formulierung) dispergiert. Die hergestellte Aufschwemmung der Liposomen mit dem eingegliederten Natriumalendronat ist durch das gelbildende Mittel stabilisiert. Danach werden der Weichmacher Glyzerin und die Konservierungsstoffe zugegeben.

Der Arbeitsvorgang bei der Herstellung von Gel weist folgende Verfahrensschritte auf:
1. Vorbereitung der Bestandteile,
1.1. Auflösung des Polymerstoffs,
1.2. Herstellung der Mischung von Glyzerin mit Konservierungsstoff,
1.3. Vermengung der Hilfskomponenten: Albuminlezithin, Olivenöl, Rizinusöl (Kastoröl), ätherisches Kieferöl, Vitamine E, D, F,
2. Vermengung der hydrophilen und der hydrophoben Phasen. Die Vermengung erfolgt in einem Homogenisator bei hochdrehzahligem Betrieb und bei Raumtemperatur. Die fertige Aufschwemmung der Lipidebläschen, welche nach dem Neutralisationsverfahren erzeugt werden, wird durch die aktive Komponente ergänzt. Die Homogenisierung erfolgt bei höchster Drehzahl des Mischers im Laufe von 5 bis 10 Minuten. Danach wird der Konservierungskomplex zugegeben. Das Gemisch wird vermengt, so dass pH = 6,9 - 7,4 aufrechterhalten wird.

Der gesamte Vorgang kann in einer Anlage vom Typ PGR-2 (┌┐┌P-2) mit einem Wasserschallschwingungserzeuger vorgenommen werden. Wenn eine wirksame Vermischungs- und Homogenisierungsanlage vorhanden ist, kann zur Herstellung von Liposomen das Ultraschallbehandlungsverfahren oder das Extrusionsverfahren mit dem Extrudieren durch Membranfilter angewendet werden.

Die Größe und die Lagenzahl der Liposomen werden nach dem Verfahren des elektronischen Mikroskopierens oder nach dem Spektrophotometrie-Verfahren überwacht.

### Klinische und Versuchsforschungen

Die Wirksamkeit und die Sicherheit der Gelform der Bisphosphonate wurden im Rahmen von klinischen Forschungen an Freiwilligen geprüft.

Die Forschungen der pharmakologischen Zusammensetzung wurden an 62 Freiwilligen, darunter Frauen und Männer in verschiedenem Alter von 32 bis 75 Jahren, vorgenommen. Die Freiwilligen wiesen unterschiedliche gesundheitliche Zustände und verschiedene Dichte der Knochengewebe auf. Jeder der Patienten wurde klinisch im Laufe von mindestens sechs Monaten beobachtet. Der klinische Status der untersuchten Patienten wurde monatlich eingeschätzt, indem die Ist-Angaben mit den Referenzangaben verglichen wurden (bekannte Mittel und Verfahren zur Vorbeugung und Behandlung der Knochenresorption).

Im ersten Schritt, d. h. vor dem Beginn des klinischen Probiervorgangs, erhielten alle Patienten die Epikutantests mit dem Versuchsgel, um eventuelle allergische Reaktionen zu prüfen. In keinem der Fälle wurden Merkmale von Hautreizung sowie lokale oder allgemeine allergische Reaktionen beobachtet.

Die Forscher, die die klinischen Untersuchungen durchgeführt haben, sowie die untersuchten Personen selbst haben festgestellt, dass dieses Mittel sehr bequem bei der praktischen Anwendung, darunter auch bei der Massage, ist.

### Beispiel 7

Kranke B., 53 J.. Osteoporose mit Immunopathie im Hintergrund. Ständige ziehende Schmerzen in Röhrenknochen. Die Anwendung von Gel mit Natriumalendronat hat den Zustand der Kranken wesentlich verbessert. 1,5 Monate nach der Behandlung: Die Beweglichkeit der Gelenke nahm zu, Ödeme und Schmerzen ließen nach, die Morgensteifheit war geringer ausgeprägt.

### Beispiel 8

Kranker G., 66 Jahre. Diagnose: Gonarthrose rechts. Nach der Anwendung von Gel mit Natriumalendronat im Laufe von drei Monaten wurde der Zustand des Patienten wesentlich besser. Das Schmerzsyndrom ließ wesentlich nach. Die Bewegungsfreiheit und -menge im Kniegelenk nahmen deutlich zu. Die Steigerung der Knochengewebedichte und die Verminderung der Osteoporose-Erscheinungen wurden durch eine Dichtemessung nachgewiesen.

### Beispiel 9

Kranke A., 55 Jahre. Diagnose: Osteoporose in der Postmenopause. Um den Zustand zu erleichtern, wurde Gel mit Natriumalendronat verordnet. Die epikutanen Applikationen wurden täglich im Laufe von drei Monaten durchgeführt. Während der Kontrolluntersuchung der Patientin nach drei Monaten wurden positive Änderungen auf dem Densitogramm, eine Erweiterung der Bewegungsfreiheit und -menge in den Knie- und Hüftgelenken sowie eine Steigerung der Belastungstoleranz festgestellt.

Die experimentellen Forschungen der Pharmakokinetik der liposomenhaltigen pharmakologischen Zusammensetzung mit Natriumalendronat in vivo wurden im Forschungsinstitut (staatliche Einrichtung des wissenschaftlichen V.V. Sakussov-Forschungsinstituts für Pharmakologie der Russischen Akademie für medizinische Wissenschaften) unter Einsatz des Radioisotop-Verfahrens an 152 gewöhnlichen (rassenlosen) Weißratten durchgeführt. Das Gewicht einer Ratte betrug ca. 180 -220 g. Das Natriumalendronat mit einer spezifischen Radioaktivität von 30 µCi/mg wurde mit Tritium markiert. Eine Dosis von Natriumalendronat von 1 mg wurde für eine Intussuszeption und für eine epikutane Applikation in Form von Gel benutzt. Das Natriumalendronat mit der radioaktiven Marke als Bestandteil von Gel befand sich in Liposomen.

Die Ergebnisse der Versuche sind in Fig. 1 und 2 dargestellt. Der Gehalt der markierten Produkte wurde während der Versuche im Plasma, im Muskel- und Knochengewebe der Ratten sowie im Harn und in den Exkrementen bestimmt. Die Proben wurden nach 2, 4, 8, 12, 24, 48 und 72 Stunden nach der Intussuszeption des Präparats und nach der epikutanen Applikation von Gel auf die enthaarte (epilierte) innere Oberfläche der Schenkel der Labortiere ausgewertet.

Nach Auswertung der Versuche wurde Folgendes festgestellt (die Ergebnisse sind in Fig. 1 und 2 dargestellt):
- die vorgeschlagene Zusammensetzung bezieht sich auf die «langlebigen» Kompositionen und wird im Körper der Ratten im Laufe von 72 Stunden ermittelt;
- die relative biologische Zugänglichkeit (Verfügbarkeit) von Natriumalendronat in der Gelform nach seinem fünfmaligen Auftragen auf die Haut von Ratten ist 2,63 mal höher als beim einmaligen Auftragen und 1,46 mal höher gegenüber der Intussuszeption;
- das epikutane Auftragen von Natriumalendronat in Form von Gel bedingt die zweifache Verlängerung der Halbeliminationszeit des Präparats gegenüber der oralen Verabreichung.

Als Ergebnis ermöglichen es die Zusammensetzung und die Verfahren zur transdermalen Lieferung, eine ausreichend hohe Konzentration von Natriumalendronat in den Geweben und in den Knochen unmittelbar im Behandlungsbereich sicherzustellen, ohne dass eine zusätzliche medizinische Ausrüstung oder ein technisches Verfahren (Iontophorese, Elektrophorese, Einreibungen mittels Massagen, Injektionen des Präparats) angewendet werden müssen.

Dabei wurden eine pharmazeutische Zusammensetzung von Natriumalendronat in Form von Gel und ein Verfahren zu seiner transdermalen Applikation entwickelt. Das Natriumalendronat oder andere Bisphosphonate befindet sich im Gel als Bestandteile von Liposomen, die eine biologische Zugänglichkeit (die Verfügbarkeit) von Natriumalendronat wesentlich verbessern. Darüber hinaus schaffen sie die Bedingungen für eine langfristige lokale Aufrechterhaltung der therapeutischen Konzentrationen von Natriumalendronat in den Stellen der Knochenresorption unterschiedlicher Ätiologie, pathologischen Frakturen und bei anderen Zuständen, bei denen die Wiederherstellung von Knochengewebe erforderlich ist.

Die vorliegende Erfindung ist mit Hilfe von Mehrfunktionsanlagen realisiert, die bei der Gewerbeherstellung von weichen Arzneiformen weit verbreitet sind.

## Patentansprüche

1. Pharmazeutische Zusammensetzung in Form eines Gels zur Vorbeugung und Behandlung von Osteoporose und anderen Zuständen, bei denen eine Wiederherstellung von Knochengewebe erforderlich ist, wobei die pharmazeutische Zusammensetzung Bisphosphonat als wirksamen Bestandteil aufweist,
**dadurch gekennzeichnet,**
**dass** das Bisphosphonat in Phospholipide-Bläschen eingegliedert ist, wobei die Phospholipide-Bläschen aus lipide- und wasseraufnehmenden Phasen gebildet sind, welche Komponenten in folgendem Komponentenverhältnis (Gew. %) umfassen:
| | |
|---|---|
| Bisphosphonat | 0,01-2,0 |
| Albuminlezithin | 1,0 - 6,0 |
| ätherisches Kieferöl | 0,05 - 0,2 |
| Kampferöl | 0,01 - 1,0 |
| Olivenöl | 0,01 - 5,0 |
| Vitamin E | 0,01 - 0,15 |
| Vitamin D | 0,01 - 0,2 |
| Carbopol | 0,4 - 0,6 |
| NaOH | 0,42 |
| Glyzerin | 2,0 - 4,0 |
| Nipagin | 0,3 |
| Nipasol | 0,1 |
| Wasser | Rest. |

2. Pharmazeutische Zusammensetzung in Form eines Gels nach Anspruch 1,
**dadurch gekennzeichnet,**
**dass** das Bisphosphonat aus folgender Gruppe gewählt ist: Alendronat, Risedronat, Etidronat, Clodronat, Pamidronat.

3. Pharmazeutische Zusammensetzung in Form eines Gels nach Anspruch 1,
**dadurch gekennzeichnet,**
**dass** die Größen der bisphosphonathaltigen Phospholipide-Bläschen im Bereich von 50 bis 250 nm liegen.

4. Verfahren zur Behandlung von Knochengeweberesorption unterschiedlicher Ätiologie,
**dadurch gekennzeichnet,**
**dass** die pharmazeutische Zusammensetzung in Form eines Gels nach Anspruch 1 epikutan in der Projektion der durch Diagnose festgestellten Resorption oder der Destruktion der Knochengewebe aufgetragen wird.

5. Verfahren zur Behandlung von Osteoporosen und Frakturen unterschiedlicher Ätiologie bei Patienten, die an Krankheiten des Magen-Darm-Kanals leiden,
**dadurch gekennzeichnet,**
**dass** die Bisphosphonate als Bestandteile von Liposomen-Gel nach Anspruch 1 transdermal aufgetragen werden, was eine Entwicklung von Komplikationen ausschließt, welche mit den Schädigungen der Schleimhäute des Magen-Darm-Kanals bei einer Intussuszeption der Bisphosphonate zusammenhängen.
